Europäisches Patentamt

European Patent Office

Office européen des brevets

(19)

(11) Publication number: **0 420 222 A1**

# EUROPEAN PATENT APPLICATION

(21) Application number: 90118518.1

(22) Date of filing: 27.09.90

(51) Int. Cl.5: **C12N 15/12**, C12P 21/02, C12N 1/21, //(C12N1/21, C12R1:19)

The applicant has filed a statement in accordance with Rule 28 (4) EPC (issue of a sample only to an expert). Accession number(s) of the deposit(s): FERM BP-2601, 3039 and 3040.

(30) Priority: 29.09.89 JP 256192/89
22.06.90 JP 165116/90
10.08.90 JP 212197/90

(43) Date of publication of application:
03.04.91 Bulletin 91/14

(84) Designated Contracting States:
AT BE CH DE DK ES FR GB GR IT LI LU NL SE

(71) Applicant: **TAKEDA CHEMICAL INDUSTRIES, LTD.**
**3-6, Doshomachi 2-chome Chuo-ku Osaka(JP)**

(72) Inventor: **Senoo, Masaharu**
**B-212, 744-1, Nishi-izumigaoka 2-chome Toyonaka, Osaka 560(JP)**
Inventor: **Watanabe, Tatsuya**
**50-1, Yamadaminami Suita, Osaka 565(JP)**
Inventor: **Igarashi, Koichi**
**66-3, Shimogamo-miyazakicho Sakyo-ku, Kyoto 606(JP)**

(74) Representative: **von Kreisler, Alek, Dipl.-Chem. et al**
**Patentanwälte Von Kreisler-Selting-Werner, Deichmannhaus am Hauptbahnhof W-5000 Köln 1(DE)**

(54) Mutein of acidic FGF, and its production.

(57) Disclosed herein are methods and means of producing novel deletion-type aFGF muteins, useful in the treatment of lesions, thrombosis and various diseases, by genetic engineering using parental aFGF genes.

## MUTEIN OF ACIDIC FGF, AND ITS PRODUCTION

FIELD OF THE INVENTION

The present invention relates to deletion-type muteins of acidic fibroblast growth factor (also, hereinafter briefly referred to as aFGF), which may be employed as wound healing promoting agents, and their production through recombinant DNA techniques.

BACKGROUND OF THE INVENTION

Acidic fibroblast growth factors are a group of endothelial cell growth factors that have been observed in hypothalamus, brain, retina, etc. and they have a molecular weight of about 16000 and an isoelectric point of 5 to 7. They are characterized by the ability to bind strongly for heparin, and in general well known as a neovascularizing factor.

Methods for the preparation of aFGF by use of genetic engineering techniques have been reported in Biotechnology, 5, 960 (1987); and Journal of Biological Chemistry, 263, 16471 (1988).

Further, an amino acid substituted mutein is disclosed in ICSU Short Reports Volume 8, Advances in Gene Technology; Protein Engineering and Production, Proceedings of the 1988 Miami Bio/Technology Winter Symposium, IRL Press, page 110.

SUMMARY OF THE INVENTION

In general, it is said that an entire structure or sequence of a mature protein is not always necessary for the expression of biological and physiological activities. It has been known that deletions of an unnecessary peptide fragment and/or a domain which inhibits a biological action will rarely result in a protein or peptide with a superior property as a physiologically active substance.

The present inventors hope, however, that through the modification of aFGF to deletion-type muteins, the present inventors will obtain a molecule which has good stability, productive efficacy, enhanced cell growth activity, and further unknown biological action which is activated.

The present inventors made extensive researches in order to prepare deletion-type muteins of aFGF by recombinant DNA techniques, and made investigations to improve its stability, improve its productive efficacy in cells, and modify its biological action. As a result of this reseach, the present inventors have completed the present invention.

It is accordingly one object of the present invention to provide deletion-type mute ins which have continuous polypeptides consisting of from 90 to 133 amino acid residues of aFGF.

It is a further aspect of the invention to provide recombinant DNAs which comprise a base sequence encoding deletion-type muteins having continuous polypeptides consisting of from 90 to 133 amino acid residues of aFGF.

It is a still further aspect of the invention to provide vectors which comprise recombinant DNAs carrying a base sequence encoding deletion-type muteins with continuous polypeptides consisting of from 90 to 133 amino acid residues of aFGF.

It is another object of the invention to provide transformants which carry vectors for the expression of a base sequence encoding deletion-type muteins with continuous polypeptides consisting of from 90 to 133 amino acid residues of aFGF.

A further aspect of the invention is to provide methods for the production of deletion-type muteins with continuous polypeptides consisting of from 90 to 133 amino acid residues of aFGF, which comprises culturing transformants which carry vectors for the expression of a base sequence encoding deletion-type muteins with continuous polypeptides consisting of from 90 to 133 amino acid residues of aFGF, in a medium under conditions suitable for the expression of the muteins.

In accordance with the present invention, it is provided that the deletion-type muteins will exhibit a greater degree of aFGF activity and other properties. In preferred embodiments of the present invention, such deletion-type muteins will exhibit enhanced various characteristics relative to the original aFGF proteins. BRIEF DESCRIPTION OF THE DRAWINGS

FIG. 1 depicts a cDNA sequence of human aFGF (haFGF) used in Example 1.

FIG. 2 shows a schematic illustration for construction of plasmid pTB1070 for the expression of 43 amino terminal residue-deleted haFGF mutein obtained in Example 1.

FIG. 3 depicts an elution profile from a Q Sepharose column used for purification of 43 amino terminal residue-deleted haFGF mute in obtained in Example 1.

FIG. 4 depicts an amino acid sequence of 43 amino terminal residue-deleted haFGF mute in obtained in Example 1. In FIG.4, M at the N terminus stands for methionine derived from the intiation codon.

FIG. 5 shows a schematic illustration for construction of plasmids pTB1207 and pTB1208 obtained in Example 2(a).

FIG. 6 shows a schematic illustration for construction of plasmid pTB1209 obtained in Example 2(a).

FIG. 7 shows a schematic illustration for construction of plasmid pTB1210 obtained in Example 2(a).

FIG. 8 depicts an amino acid sequence of 9 amino terminal residue-deleted haFGF mute in obtained in Example 2. In FIG.8, M at the N terminus stands for methionine derived from the intiation codon.

FIG. 9 depicts an amino acid sequence of 12 amino terminal residue-deleted haFGF mutein obtained in Example 2. In FIG. 9, M at the N terminus stands for methionine derived from the intiation codon.

## DESCRIPTION OF THE PREFERRED EMBODIMENTS

The mature aFGF which is the parent of the muteins according to the present invention includes a bovine aFGF, a human aFGF and the like. The amino acid sequences of bovine and human aFGF are disclosed in Biochemical and Biophysical Research Communications Vol.138, No. 2, pp. 611-617, (1986). Among them, human aFGF is preferred.

The muteins according to the present invention are those comprising continuous polypeptides consisting of from 90 to 133 amino acid residues of aFGF.

The muteins according to the present invention are more preferably those comprising continuous polypeptides consisting of from 120 to 131 amino acid residues of aFGF, still more preferably those comprising continuous polypeptides consisting of from 125 to 131 amino acid residues of aFGF. Further, the muteins according to the present invention are more preferably those comprising continuous polypeptides consisting of from 131 to 133 amino acid residues of aFGF.

The muteins according to the present invention are preferably those lacking amino acids from the N-terminus of mature aFGF. Such deletion-type muteins can lack up to 3 amino acids from the C-terminus of mature aFGF.

Examples of said deletion-type muteins may include, for example, those lacking any of 8 amino acids, 9 amino acids, 11 amino acids, 12 amino acids, 20 amino acids, and 43 amino acids from the N-terminus of human aFGF.

For the preparation of said muteins, conventional recombinant DNA techniques as well as a site-directed mutagenesis is applicable and useful. This site-directed mutagenesis technique is well known in the literature, for example, Lather, R. F. and Lecoq, J. P., Genetic Engineering, Academic Press, (1983), 31-50. The mutagenesis directed on oligonucleotides is described in Smith, M. and Gillam, S., Genetic Engineering; Principle and Method, Plenum Press, (1981), Vol.3, 1-32.

The structual genes encoding the muteins may be prepared by, for example,

(a) hybridizing a single-stranded DNA comprising a single strand of aFGF structual gene, with an oligonucleotide primer having mutation

(b) elongating the primer with DNA polymerase to form a heteroduplex having the mutation, and

(c) replicating the heteroduplex with the mutation.

The sizes of oligonucleotide primer are determined depending on conditions needed for stable hybridization of the primer with the gene region to be mutated and capability of oligonucleotide synthesis presently available. Upon the design of the mutagenic oligonucleotide, factors which should be considered (e. g. total size, number of nucleotides except for mismatched one for the mutation) are described in Smith, M. and Gillam, S. (supra ). Typically, the total length of oligonucleotide is adjusted to such length that stable and unique hybridization at the mutation site is optimized and the extensions from the mutation site to the 5' and 3' termini should be long enough to prevent correction of the mismatches by the exonuclease activity present in DNA polymerase.

The size of oligonucleotide primer used depends upon conditions necessary for stable hybridization of the primer to the gene region to which mutation is to be introduced. Limitations in currently available methods of oligonucleotide synthesis also affect which size is preferable. The factors to be considered in designing oligonucleotide intended for the use for mutagenesis directed by the oligonucleotide (e.g. the overall size of the nucleotide and the size of the mismatching portion at the mutation site) are well known and, for example, are described by Smith, M. and Gillam, S. in the above-mentioned literature. In general, the overall length of the oligonucleotide is adjusted to such length that stable and unique hybridization at the mutation site is optimized, and the extensions between the mutation site and the 5'- and 3'-terminals are provided with sufficient sizes to prevent mutation induced by the exonuclease activity of DNA polymerase.

The oligonucleotides used for mutagenesis in accordance with the present invention normally contain some 12 to 24 bases, preferably 14 to 20 bases, and more preferably 14 to 18 bases. These normally contain at least about 3 base 3'-terminal of the codons to be changed.

For the purpose of obtaining, for example, a mutein lacking aFGF-constituent amino acids, a mutagenic aFGF gene may be produced in 3 different ways. In one of the 3 cases, the amino terminal of aFGF is deleted; in another case, a central region of aFGF is deleted; and in the remaining one case, the carboxyl terminal is deleted.

In the case of deletion of the amino terminal, a codon of the gene which encodes the carboxyl terminal of the amino acid sequence to be deleted is changed to the Met-encoding codon ATG by site-directed mutagenesis, and the codon has an appropriate restriction enzyme recognition site produced in the 5'-terminal and 3'-terminal sides of the gene which encodes the sequence to be deleted, and the relevant gene is cleaved off from the gene by enzymatic digestion, and this is followed by re-ligation of the remaining 2 gene fragments to construct the desired gene which encodes aFGF lacking the specified amino acids. It is of course necessary not to shift the reading frame due to the digestion with the restriction enzymes.

In the case of deletion of an amino acid sequence in the carboxyl terminal side, a codon of the gene which encodes amino-terminal amino acids of the sequence to be deleted is changed to a stop codon by site-directed mutagenesis.

The design of oligonucleotide primer of course varies with which amino acid is to be changed and can be accomplished readily by the skilled artisan.

The primer is hybridized to a single-stranded phage resulting from cloning of a single-stranded of the aFGF gene, such as M13 [Yanish-Perror, C. Vieira, and J. Messing, Gene, 33, 103-119 (1985); Messing, J., Methods in Enzymology, 101, 20-78 (1983)], fd [R. Herrman et al., Molecular and General Genetics, 177, 231 (1980)] or $\phi \times 174$ [M. Smith and S. Gillam, Genetic Engineering, Plenum Press, Vol. 3, pp. 1-32 (1981)]. It is noted that the phage is capable of carrying either the sense chain or antisense chain of the gene. When the phage carries an antisense chain, in addition to discrepancies from the relevant codon determining a triplet which has encoded another amino acid, the primer may have discrepancies due to codon degeneracy from the sense chain region containing the codon to which mutation is to be induced. Similarly, when the phage carries a sense chain, the primer may not be complementary to the sense chain region containing the codon to which mutation is to be induced, as well as appropriate discrepancies from the triplet which pairs to the codon to be deleted. The conditions used for hybridization are described by Smith, M. and Gillam, S. in the above-mentioned literature. Temperature is normally between about 0°C and about 70°C, more generally, between about 10°C and about 50°C. After hybridization, the primer is elongated on the phage DNA by reaction with Escherichia coli DNA polymerase I, T4 DNA polymerase, reverse transcriptase or other appropriate DNA polymerase. The resulting dsDNA is converted to a closed circular dsDNA by treatment with DNA ligase such as T4 DNA ligase. The DNA molecules containing single-stranded regions can be decomposed by S1 endonuclease treatment.

The resulting mutational heteroduplex is used to transform an infectable host organism or cell. In the replication of the heteroduplex by the host, progenies are produced from both chains. Following the replication, the mutant gene is isolated from a progeny of the mutant chain, and is inserted in an appropriate vector, which is then used to transform an appropriate host organism or cell.

The phage DNA carrying the mutational gene is then isolated, and incorporated in a plasmid.

Plasmids used for the invention may include, for example, pBR322 [Gene, 2 , 95 (1977)], pBR325 [Gene, 4 , 121 (1978)], pUC12 [Gene, 19 , 259 (1982)], and pUC13 [Gene, 19 , 259 (1982)] derived from E. coli , pUB110 [Biochemical and Biophysical Research Communications Vol. 112 , 678 (1983)], and the like. Other plasmids can be used as long as they are replicable and maintainable in hosts.

Methods for recombination of plasmid include those described in T. Maniatis, et al., Molecular Cloning, Cold Spring Harbor Laboratory, 239 (1982), etc.

The cloned genes are ligated downstream from promoters in a suitable vehicles (vectors) for expression to obtain expression vectors.

Vectors used in the invention may include plasmids derived from E. coli as above-mentioned (e.g. pBR322 , pBR325 , pUC12 , and pUC13), plasmids derived from Bacilli (e.g. pUB110, pTP5, and pC194), plasmids derived from yeast (e.g. pSH19, and pSH15), bacteriophages such as bacteriophage λ , animal viruses such as retroviruse and vaccinia viruse, and insect viruses.

The cloned genes may contain an initiation codon, ATG at 5' end and further a termination codon, TAA, TGA or TAG at 3' end. For the purpose of expression of the gene, the promoters are ligated upstream from the genes. The promoters used for the invention may be any of promoters suitable for hosts which are used to express genes.

Upon transformation, when the host is E. coli , trp promoters, lac promoters, recA promoters, λ pL promoters, lpp promoters, T7 promoters, and the like are preferred, when the host is Bacillus subtilis SP01 promoters, SP02 promoters, penP promoters, and the like and when the host is yeast , PH05 promoters, PGK promoters, GAP promoters, ADH promoters, and the like. The most preferable host is E. coli , the most preferable promoter is the trp promoter or the T7 promoter.

When the host is an animal cell, promoters include those derived from SV40, promoters of retroviruses, and the like, and the most preferred promoter is one of those derived from SV40.

By using the thus obtained vector which comprises recombinant DNA having the base sequence encoding the mutein, transformants which carry the vector are prepared.

Hosts used for transformants in the invention may include, for example, Escherichia coli , Bacillus subtilis , yeast, animal cells, insect cells, and the like.

Examples of the above-mentioned Escherichia coli include Escherichia coli K12 DH1 (Proc. Natl.Acad. Sci. USA, 60 , 160 (1968)), E. coli JM103 (Nucleic Acids Research, 9 , 309 (1981)), E. coli JA221 (Journal of Molecular Biology, 120 , 517 (1978)), E. coli HB101 (Journal of Molecular Biology, 41, 459 (1969)), E. coli C600 (Genetics, 39 , 440 (1954)), E. coli MM294 (Proc. Natl.Acad. Sci. USA, 73 , 4174 (1976)), and the like.

The above-mentioned Bacillus subtilis include, for example, Bacillus subtilis MI114 (Gene, 24 , 255 (1983)), B. subtili s 207-21 (Journal of Biochemistry, 95 , 87 (1984)), and the like.

The above-mentioned yeasts include, for example, the genus Saccharomyces , Saccharomyces cerevisiae AH22R⁻,S. cerevisiae NA87-11A, S. cerevisiae DKD-5D, and the like.

The above-mentioned animal cells include, for example, COS-7 monkey cells, Vero, Chinese hamster cells (CHO), mouse L cells, human FL cells, and the like.

The transformation of the E. coli is conducted according to conventional methods such as the methods disclosed in Proc. Natl. Acad. Sci. U.S.A., 69 , 2110 (1972), Gene, 17 , 107 (1982), etc.

The transformation of the Bacillus subtilis is conducted according to conventional methods such as the methods disclosed in Molecular & General Genetics, 168, 111 (1979), etc.

The transformation of the yeast is conducted according to conventional methods such as the methods disclosed in Proc. Natl. Acad. Sci. U.S.A., 75 , 1929 (1978), etc.

The transformation of the animal cell is conducted according to conventional methods such as the methods disclosed in Virology, 52 , 456 (1973), etc.

The transformants are thus obtained which carry the vectors comprising the recombinant DNA having the base sequences coding for the muteins.

The muteins according to the present invention are produced through cultivating the transformant in a medium under conditions suitable for the expression of the mutein.

Upon the cultivation of the transformant where the host is selected from Escherichia coli or Bacillus subtilis , a liquid medium is suitable as a medium used for cultivation. The liquid medium may contain a carbon source, a nitrogen source, minerals and the like, which are necessary for the growth of the transformants.

The carbon source may include, for example, glucose, dextrin, soluble starch, sucrose, etc. The nitrogen source may include, for example, inorganic or organic materials such as ammonium salts, nitrate salts, cornsteep liquor, peptone, casein, meat extracts, soy bean cakes, potato extracts, etc. The mineral may include, for example, calcium chloride, sodium dihydrogenphosphate, magnesium chloride, etc. Further yeast extracts, vitamins, growth promoting factors, etc. may be supplemented. The pH of medium can vary preferably from about 6 to about 8. The medium used for culturing the transformant E. coli includes preferably, for example, M9 medium containing glucose and casamino acid [Miller, Journal of Experiments in Molecular Genetics, 431-433, Cold Spring Habor Laboratory, New York (1972)]. According to necessity, for working the promoter efficiently, reagents such as, for example, 3 β-indolyl acrylic acid and isopropyl thiogalactopyranoside may be added.

Where the host is Escherichia coli , the cultivation of the transformant can be carried out usually at temperature of from about 15 to 43° C for about 3 to about 24 hours, and as the case may be, aeration and/or agitation may also be added.

Where the host is Bacillus subtilis , the cultivation of the transformant can be carried out usually at temperature of from about 30 to 40° C for about 6 to about 24 hours, and as the case may be, aeration and/or agitation may also be added.

Upon the cultivation of the transformant where the host is yeast, the medium used includes preferably,

5

for example, Burkholder minimum essential medium [Bostian, K. L., et al., Proc. Natl. Acad. Sci. U.S.A., 77 , 4505 (1980)], etc. The pH of medium can be preferably adjusted to ranges from about 5 to about 8. The cultivation of the transformant can be carried out usually at temperature of from about 20 to 35° C for about 24 to about 72 hours, and as the case may be, aeration and/or agitation may also be added.

Upon the cultivation of the transformant where the host is an animal cell, the medium used includes preferably, for example, MEM medium supplemented with about 5 % to 20 % fetal calf serum [Science,122, 501 (1952)], DMEM medium [Virology, 8 , 396 (1959)], RPMI1640 medium [Journal of the American Medical Association,, 199, 519 (1967)], 199 medium [Proceeding of the Society for the Biological Medicine, 73 , 1 (1950)], etc. The pH of medium can vary preferably from about 6 to about 8. The cultivation of the transformant can be carried out usually at temperature of from about 30 to 40° C for about 15 to about 60 hours, and as the case may be, aeration and/or agitation may also be added.

The isolation and purification of the aFGF mutein from the culture can be conducted, for example, according to the following method.

Methods for extracting the aFGF mutein from the cultured micro-organisms or cells, may include a method comprising collecting the micro-organisms or cells in a conventional manner after cultivation, and then eluting the subject protein out of the cell by suspending the micro-organisms or cells in a buffer containing protein denaturing agents such as guanidine hydrochloride, a method comprising disrupting the micro-organism or the cell by french press, sonication, lysozyme, and/or freezing-thawing and then obtaining the aFGF mutein by centrifugation, etc. Particulary preferable are the french press method and the combined method of lysozyme and sonication.

The purification of the aFGF mutein from the supernatant can be conducted by combining suitably known isolation and purification methods well known to the skilled artisan. The isolation and purification method may include a method utilizing solubilities such as salting-out, solvent precipitation and the like, a method utilizing mainly differences of molecular weight such as dialysis, ultrafiltration, gel filtration and SDS-poly acrylamide gel electrophoresis and the like, a method utilizing differences of charge such as ion-exchange chromatography and the like, a method utilizing specific affinities such as affinity chromatography and the like, a method utilizing differences of hydrophobicity such as reversed phase high-performance liquid chromatography and the like, a method by the use of differences of isoelectric point such as isoelectric electrophoresis and the like.

More specifically, contaminating nucleic acids, acidic proteins, etc. can be removed by subjecting the above-mentioned supernatant to ion exchange chromatography using DEAE cellulose and the like as a carrier. For example, it is advantageous that the supernatant is applied to a DEAE cellulose column equilibrated with a buffer such as Tris at neutral or near pH to collect pass-through fractions.

The application of the aFGF mutein in the extracts to affinity chromatography employing heparin-Sepharose as a carrier is advantageous among the methods for purification of aFGF mutein. Purification of aFGF mutein can be conducted, for example, by loading the eluate on a heparin Sepharose column equilibrated with a buffer such as Tris and phosphate at neutral or near pH, followed by elution with a linear gradient such as NaCl and the like after sufficient washing.

Particularly useful is a heparin column which has been developed for high performance liquid chromatography (for example, Shodex AF-pak HR. 894, Showadenko K. K., Japan). In a similar fashion of the heparin Sepharose column, the aFGF mutein can be recovered as an approximately homogeneous product by loading the sample thereon in a neutral or near buffer followed by elution with a linear gradient such as NaCl and the like after sufficient washing. The product thus obtained can be subjected to dialysis and lyophilization to give a dry powder. Preferred is the preservation of the lyophilized product by adding serum albumin and the like as a stabilizer since adsorption of the product to the container wall can thereby be prevented.

During purification or preservation processes, the presence of a slight amount of a reducing agent in admixture therewith is useful for prevention of oxidation thereof. The reducing agents may include $\beta$-mercaptoethanol, dithiothreitol, glutathione and the like.

The present mutein of aFGF thus obtained has a higher stability and more increased activity than the matured aFGF, and therefore it can be advantageously used as therapeutic agents for burns, wounds, thrombosis, arteriosclerosis, etc. Furthermore since the mutein of the present invention possesses growth promoting activity for nerve cells, it is useful in treating various neuropathies.

For its pharmaceutical use, the aFGF muteins can be safely administered to warm-blooded mammals (e.g. humans, mice, rats, hamsters, rabbits, dogs, cats) parenterally or orally either in a powder form per se or in the form of pharmaceutical compositions (e,g, injections, tablets, capsules, solutions,ointments) in admixture with pharmaceutical acceptable carriers, excipients and/or diluents. The pharmaceutical compositions can be formulated in accordance with a conventional method.

When used for the above pharmaceutical purposes, the aFGF muteins are administered, for example, to the above warm-blooded mammals in an appropriate amount selected from the range of from about 10 ng to 10 μg/kg body weight a day according to route of administration, reaction sensitivities, severity of the disease, etc.

Further, the aFGF mutein thus purified can be used as a reagent for promoting cell cultivation. In this instance, the aFGF protein is added to the medium preferably in an amount of about 0.1 to 10μg per liter of medium.

The following transformants which were obtained in the Examples mentioned below were deposited at the Institute for Fermentation, Osaka, Japan (IFO), and at the Fermentation Research Institute, Agency of Industrial Science and Technology, Ministry of International Trade and Industry, Japan (FRI) under the Budapest Treaty.

Their accession numbers on the deposit dates are shown in Table 1 below ( The deposit dates are indicated in parenthesis ).

TABLE 1

| Transformant | IFO | FRI |
|---|---|---|
| E.coli MM294(DE3)/pLysS, pTB 1070 (Example 1) | IFO 14938 (September 12, 1989) | FERM BP-2601 (September 20, 1989) |
| E.coli MM294(DE3)/pLysS, pTB 1209 (Example 2) | IFO 15069 (July 24, 1990) | FERM BP-3039 (August 6, 1990) |
| E.coli MM294(DE3)/pLysS, pTB 1210 (Example 2) | IFO 15070 (July 24, 1990) | FERM BP-3040 (August 6, 1990) |
| E.coli K12 MM294/pTB 917 (Example 1) | IFO 15093 (September 17, 1990) | |

In the specification and drawings of the present application, the abbreviations used for bases, amino acids and so forth are those recommended by the IUPAC-IUB Commission on Biochemical Nomenclature or those conventionally used in the art. Examples thereof are given below. Amino acids for which optical isomerism is possible are, unless otherwise specified, in the L form.

DNA: Deoxyribonucleic acid
cDNA: Complementary deoxyribonucleic acid
A: Adenine
T: Thymine
G: Guanine
C: Cytosine
RNA: Ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
Tdr: Thymidine
EDTA: Ethylenediamine tetraacetic acid
SDS: Sodium dodecyl sulfate
G, Gly: Glycine
A, Ala: Alanine
V, Val: Valine
L, Leu: Leucine
I, Ile: Isoleucine

S, Ser: Serine
T, Thr: Threonine
C, Cys: Cysteine
M, Met: Metionine
E, Glu: Glutamic acid
D, Asp: Aspartic acid
K, Lys: Lysine
R, Arg: Arginine
H, His: Histidine
F, Phe: Pheylalanine
Y, Tyr: Tyrosine
W, Trp: Tryptophan
P, Pro: Proline
N, Asn: Asparagine
Q, Gln: Glutamine

The numbering of constituent amino acids in human and bovine aFGF used therein is in accordance with that described in Biochemical and Biophysical Research Communications Vol. 138, 611-617 (1986).

## Example

The invention is further illustrated by the following examples. These examples are not intended to limit the invention in any manner.

## Example 1

### Preparation of 43 Amino Terminal Residue-Deleted Human aFGF Mutein

#### (a) Construction of Plasmid for Expression

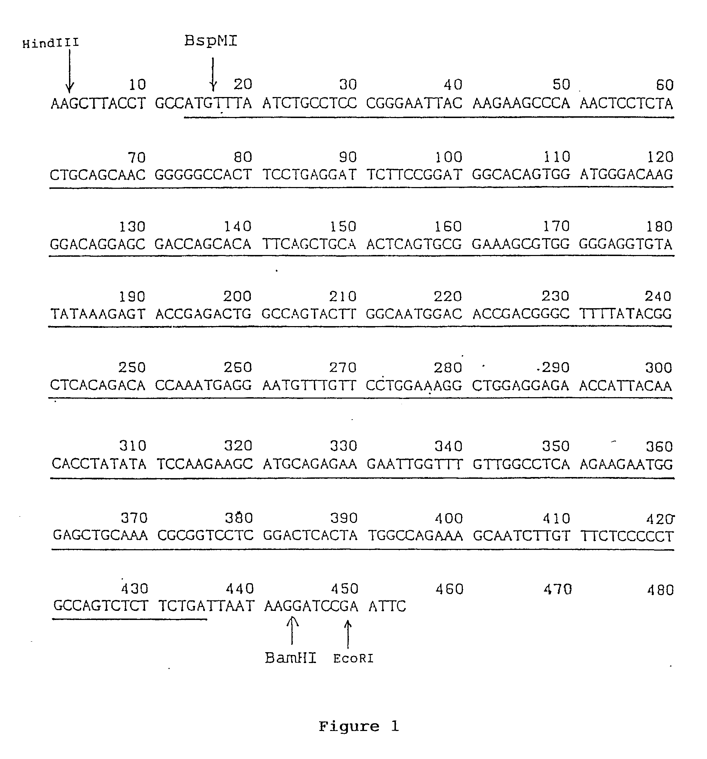

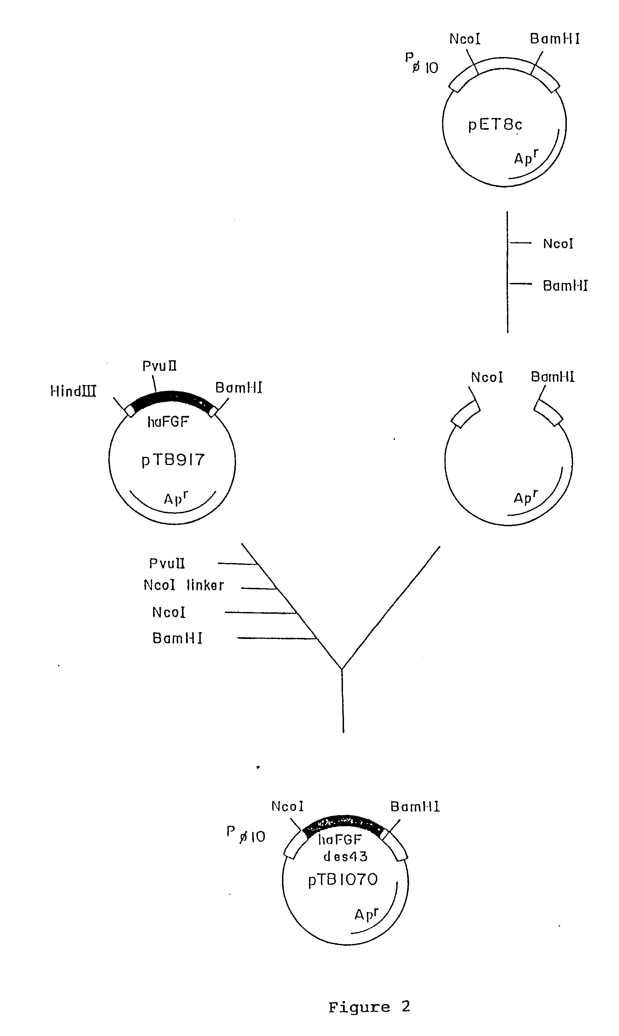

Plasmid pTB917 carrying chemically synthesized cDNA for human aFGF (Figure 1) in pUC18 [Methods in Enzymology, 101, 20-78 (1983)] was isolated from the transformant, E. coli K12 MM 294 /pTB917 (IFO 15093) according to a conventional method. The plasmid pTB917 was cleaved with Puvll (Figure 2), followed by ligation of Ncol linkers, 5'-CCATGG-3' with T4 DNA ligase. These plasmids were cleaved with Ncol and BamHI to prepare 0.3 kb DNA fragments.

Plasmid pET-8c carrying T7 phage $\phi$ 10 promoter (given by Studier, F. W. (Brookhaven National Labs U.S.A.), this pET-8c is described in J. Mol. Biol. 189, 113-130 (1986), Gene, 56 , 125-135 (1987)) was used for a vector DNA. The pET-8c was cleaved with Ncol and BamHI, followed by ligation of the 0.3 kb fragment thereto with T4 DNA ligase to obtain pTB1070 (Figure 2).

#### (b) Expression of Amino Terminal Deleted haFGF Mutein cDNA in E. coli

$\lambda$ phage DE3 having T7 phage RNA polymerase gene [Studier, F. W. et al., J. Mol. Biol.189, 113-130 (1986)] was lysogenized in E. coli MM 294 strains followed by transfection of plasmid pLysS carrying T7 phage lysozyme gene [Studier, F. W. et al., J. Mol. Biol. 189, 113-130 (1986)] to produce E. coli MM 294 (DE3)/pLysS. The E . coli was transformed with pTB1070 to give E. coli MM 294 (DE3)/pLysS, pTB1070 (IFO 14938, FERM BP-2601). The transformant was incubated in a medium containing 35 $\mu$g/ml of ampicillin and 10 $\mu$g/ml of chloramphenicol at 37 °C. When the turbidity reached to Klett 120, isopropyl $\beta$-D-thiogalactoside (IPTG) was added to finally 0.5 mM. Incubation was continued for additional two hours. The transformants were harvested by centrifugation, washed with phosphate buffered saline (PBS) cooled in ice, then recollected, and stored at -20 °C until use.

8

EP 0 420 222 A1

(c) Purification of 43 Amino Terminal Residue Deleted haFGF Mutein

E. coli MM 294 (DE3)/pLysS, PTB1070 (IFO 14938, FERM BP-2601) collected from 125 ml of the culture was suspended in 10 ml of ice-cooled 10 mM Tris-HCl (pH 7.4) containing 10 mM EDTA, 0.2 M NaCl, 10% sucrose and 0.25 mM phenylmethylsulfonyl fluoride (PMSF), followed by addition of egg white lysozyme to 0.5 mg/ml.

The suspension was allowed to stand in ice for an hour, then warmed to 37 °C for five minutes, sonicated whilst ice cooling, and centrifuged.

The precipitate was suspended in 2 M NaCl followed by recentrifugation to give a precipitate which was suspended in 15 ml of 20 mM Tris-HCl (pH 7.4) containing 6 M urea and 10 mM dithiothreitol (DTT), and incubated in ice for 3 hr. whilst intermittently stirring. The resulting solution was centrifuged to give a supernatant which was applied to a Q-Sepharose column (2.5 cm x 8 cm) equilibrated with 20 mM Tris-HCl (pH 7.4) containing 3 M urea.

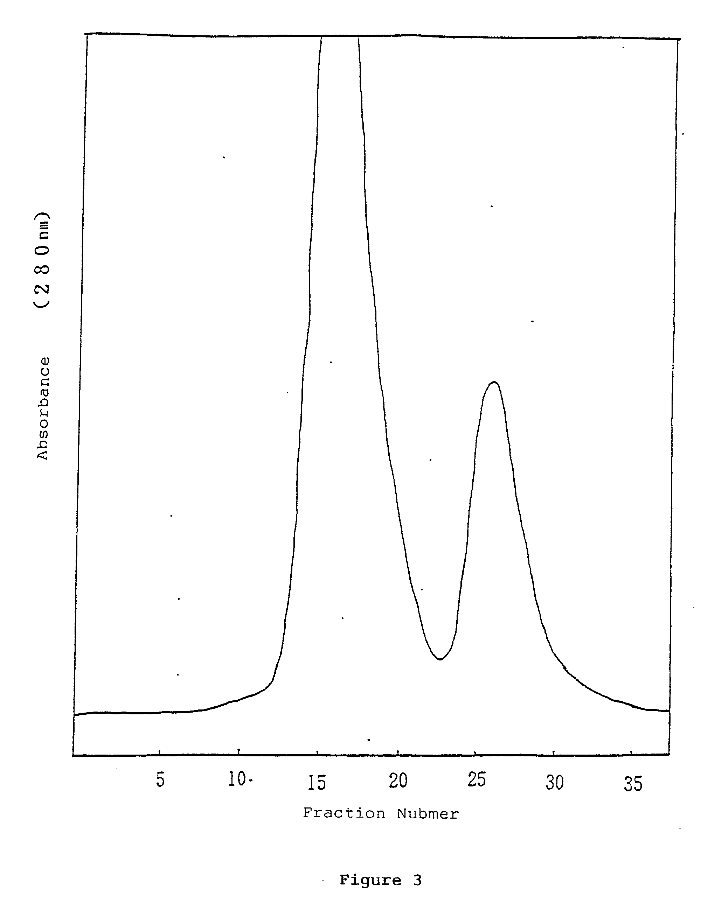

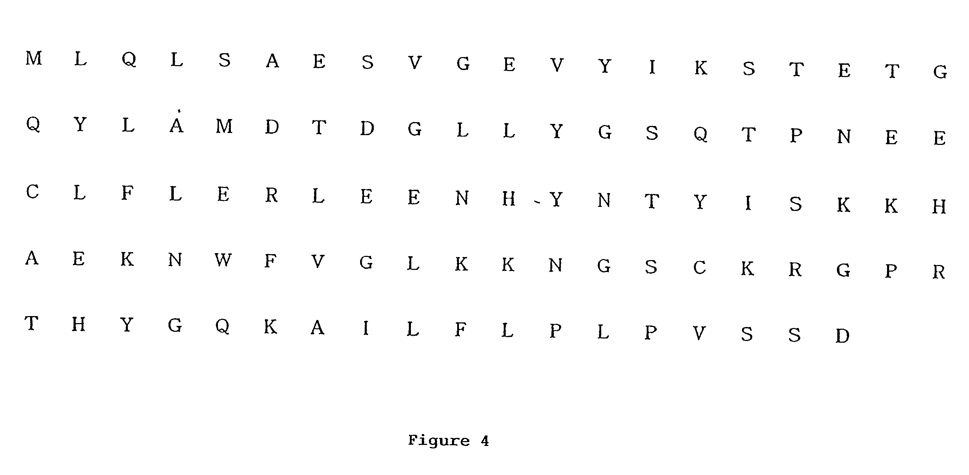

The column was washed with a buffer used for equilibrating, eluted with a linear gradient of from 0 M to 1 M NaCl at a flow rate of 0.6 ml/min. for 160 min. and 2.5 ml fractions were collected (Figure 3). The eluted fractions 14-19 were pooled and dialyzed against 2 l of 20 mM Tris-HCl (pH 7.4) containing 5 mM DTT overnight followed by 3 l of 20 mM Tris-HCl (pH 7.4) containing 1 mM DTT for 3 hr. Fourty three amino terminal residue deleted human aFGF mutein (3.2 mg) which has the amino acid sequence as depicted in FIG. 4. was obtained by the above procedures.

Example 2

Preparation of 9 Amino Terminal Residue-Deleted Human aFGF Mutein and 12 Amino Terminal Residue-Deleted Human aFGF Mutein

(a) Construction of Plasmid for Expression

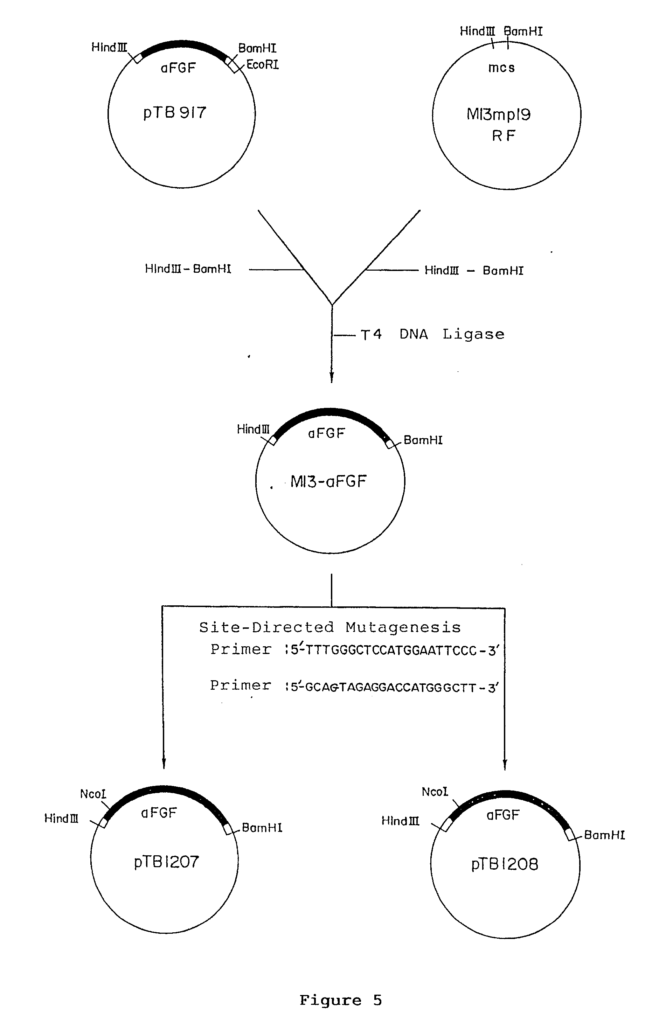

Plasmid pTB917 (Example 1(a)) inserted with human aFGF cDNA (Figure 1) was digested with HindIII and BamHI to cut out a cDNA portion which was inserted into a HindIII - BamHI site of RF DNA of M13 mp19 phages [Gene, 33 , 103-119 (1985)] with T4 DNA ligase (Figure 5). From the resulting phage the single stranded DNA was prepared into which mutagenesis was introduced by the use of primers
5′-TTTGGGCTCCATGGAATTCCC-3′ and
5′-GCAGTAGAGGACCATGGGCTT-3′ to obtain pTB 1207 and pTB1208, respectively (Figure 5). For the reaction of site-directed mutagenesis, mutagenesis kits purchased from Amarsham (U. K.) were used.

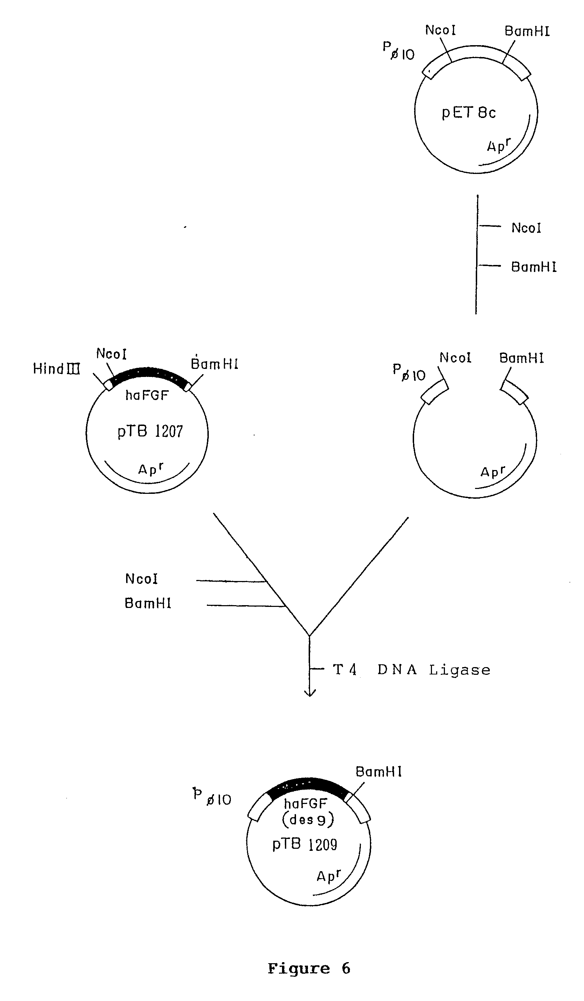

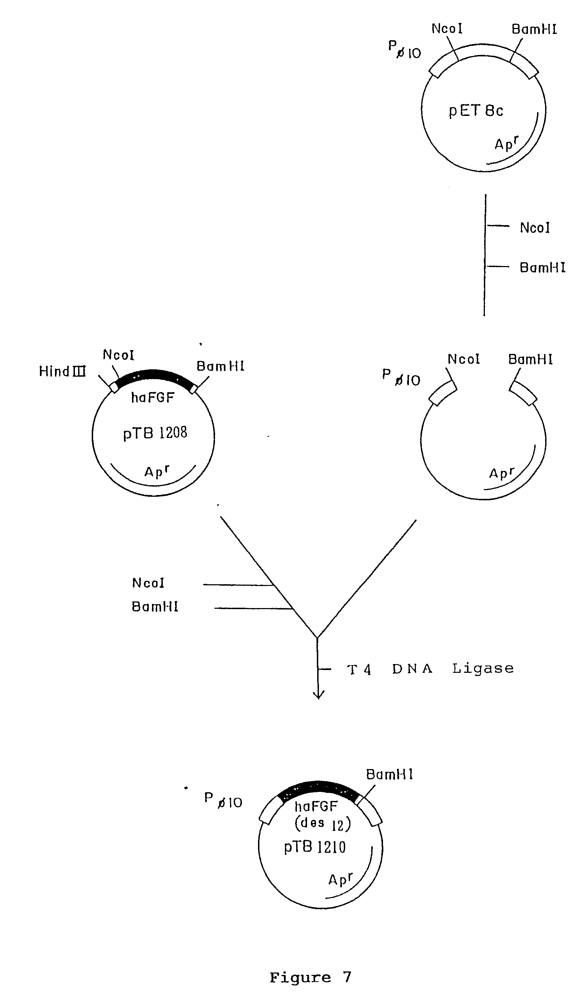

The plasmids pTB1207 and pTB1208 were digested with NcoI and BamHI to cut out mutated aFGF cDNAs which were inserted into a NcoI - BamHI site of said plasmid pET-8c carrying T7 phage φ 10 promoter (J. Mol. Biol. 189, 113-130 (1986) and Gene, 56, 125-135 (1987)) to obtain plasmids pTB 1209 for expression of 9 amino terminal residue deleted-type haFGF mutein and pTB1210 for expression of 12 amino terminal residue deleted-type haFGF mutein, respectively (Figure 6 and Figure 7).

(b) Expression of cDNA Coding for haFGF Mutein Deleted Amino Termini in E. coli

λ phage DE 3 having T7 phage RNA polymerase gene [Studier, F. W. et al., J. Mol. Biol. 189, 113-130 (1986)] was lysogenized in E. coli MM 294 followed by transfection of plasmid pLysS carrying T7 phage lysozyme gene [Studier, F. W. et al., J. Mol. Biol. 189, 113-130 (1986)] to produce E. coli MM 294 (DE3)-/pLysS.

The E. coli was transformed with pTB1209 and pTB1210 to produce E. coli MM 294 (DE3)/pLysS, pTB1209 (IFO 15069, FERM BP-3039) and E. coli MM 294 (DE3)/pLysS, pTB1210 (IFO 15070, FERM BP-3040), respectively. The transformants were cultured in a medium containing 35µg/ml of ampicillin and 10 µg/ml of chloramphenicol at 37 °C. When the turbidity reached to Klett 120, isopropyl β-D-thiogalactoside (IPTG) was added to finally 0.5 mM. Incubation was continued for additional two hours.

The transformants were harvested by centrifugation, washed with phosphate buffered saline (PBS) cooled in ice, then recollected, and stored at -70 °C until use.

A part of the resulting microorganisms was used, sonicated, and certifuged to prepared a crude extract

9

which was assayed for biological activities according to the method described below to exhibit a significant result.

## Biological Activity

Activity of human aFGF was assayed by the determination of $^3$H-thymidine incorporation into DNA of mouse BALB/c 3T3 cell lines, in accordance with the method of Sasada, et al. (Mol. Cell Biol. 8 , 588-594 (1988)). Upon addition of the sample, depending on necessity, a heparin solution (SIGMA Grade I) was added to media and the sample.

(c) Purification of 9 Amino Terminal Residue Deleted haFGF Mutein and 12 Amino Terminal Residue Deleted haFGF Mutein

E. coli MM 294 (DE3)/pLysS, pTB1209 (IFO 15069, FERM BP-3039) or E. coli MM 294 (DE3)/pLysS, pTB1210 (IFO 15070, FERM BP-3040) collected from 12.5 ml of the culture were suspended in 1 ml of ice-cooled 10 mM Tris-HCl (pH 7.4) containing 10 mM EDTA, 0.2 M NaCl, 10% sucrose and 0.25 mM phenylmethylsulfonyl fluoride (PMSF), and followed by sonication whilst ice cooling, respectively. The suspension was allowed to stand in ice for an hour, and centrifuged (SORVALL, 18K rpm, 30 min., 4° C) to obtain a supernatant. The supernatant was applied to a heparin HPLC column (0.8 x 5 cm) equilibrated in 20 mM Tris-HCl (pH 7.4). The column was washed with 20 mM Tris-HCl (pH 7.4) containing 0.6 M NaCl, and then eluted with a linear gradient of 0 M to 2 M NaCl (flow rate: 1 ml/min., for 1 hour), and fractionated.

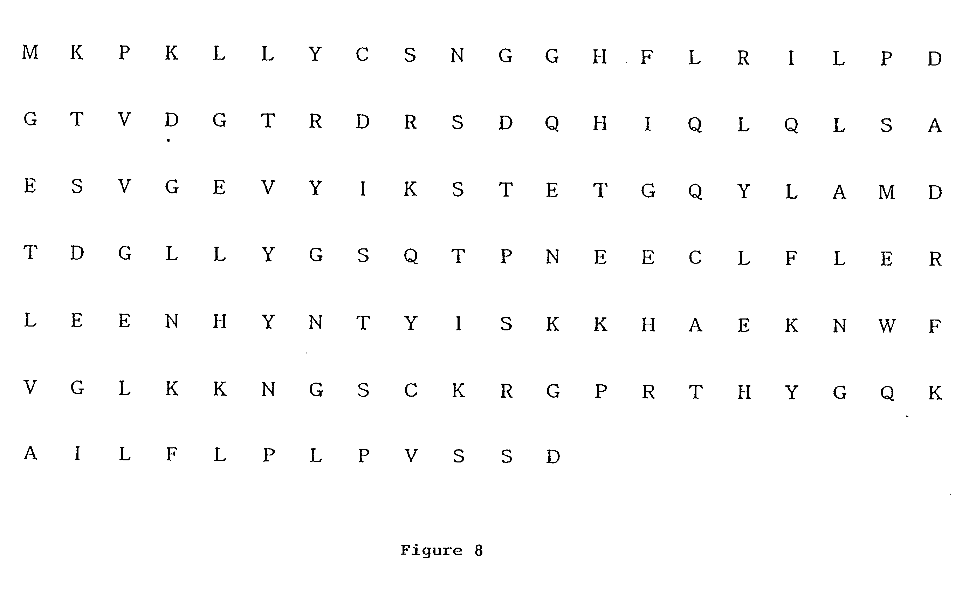

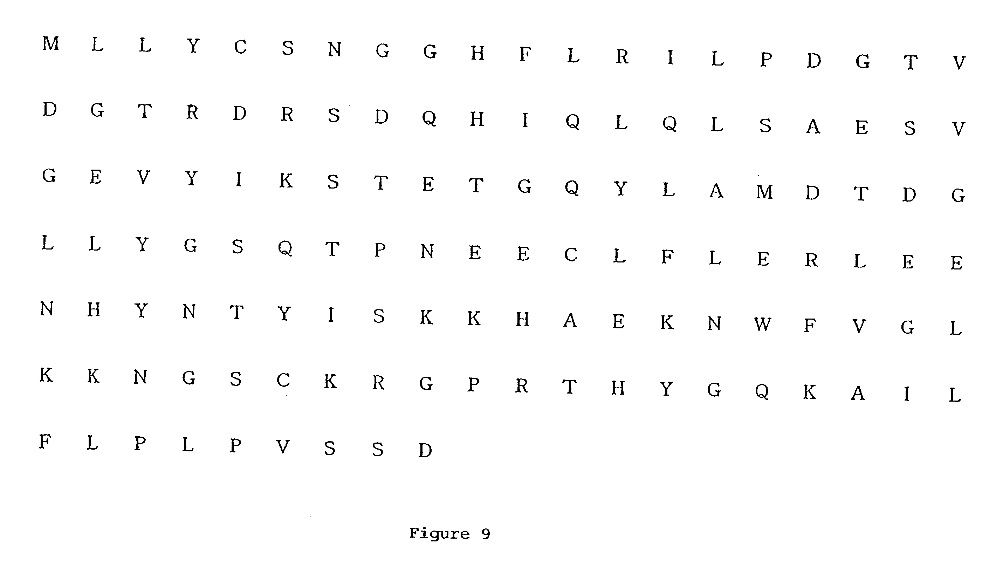

Fractions were pooled which contained deletion-type human aFGF muteins. Nine amino terminal residue deleted human aFGF mutein (1.2 mg) which has the amino acid sequence as depicted in FIG. 8. and 12 amino terminal residue deleted human aFGF mutein (0.1 mg) which has the amino acid sequence as depicted i FIG. 9. were obtained by the above procedures.

(d) Biological Activity of 9 Amino Terminal Residue Deleted haFGF Mutein and 12 Amino Terminal Residue Deleted haFGF Mutein

The biological activities of 9 amino terminal residue deleted haFGF mutein and 12 amino terminal residue deleted haFGF mutein obtained in Example 2(c) were assayed on the ability to stimulate DNA synthesis in BALB/3T3 cells maintained at a low serum concentration as reported by Sasada, et al. (Mol. Cell Biol. 8 , 588-594 (1988)), respectively. BALB/3T3 cells were plated in DMEM with 5% calf serum ($2 \times 10^3$ cells per well), and the next day, the medium was replaced with 0.2 ml of DMEM containing 0.5% calf serum. Three days later, 0.01 ml of serial 1:5 dilutions of samples (in DMEM with 0.5% crystalline bovine albumin) was added to each well with or without heparin (Sigma Grade I, final concentration: 50 $\mu$g/ml). At 18 h after the sample was added, 1 $\mu$Ci of [methyl-$^3$H]thymidine (5 Ci/mmol; CEA, France) was added. After the cells were incubated for 4 h, they were trypsinized and collected on glass filters with a Titertek cell harvester (Skatron, Lier, Norway). The filters were dried and immersed in scintillation fluid; radioactivity was measured in a scintillation counter. The activity was calculated by determining the dilution of factor required to give 50% of the maximal stimulation using bovine pituitary FGF (Takara Shuzo, Japan) as the standard. The specific activities of the muteins with or without heparin are shown in Table 2 in which the values are depicted as intact aFGF in the presence of heparin for 1.0.

TABLE 2

|  | Heparin | |
| --- | --- | --- |
|  | - | + |
| aFGF | 0.02 | 1.0 |
| des 1-9 | 0.01 | 0.7 |
| des 1-12 | <0.01 | 0.2 |

The deletion-type muteins according to the present invention are more stable and active than mature aFGF and therefore useful in treating lesions, thrombosis, arteriosclerosis, etc.

It is understood that the preceding representative examples may be varied within the scope of the present invention by one skilled in the art to achieve essentially the same results.

As many widely different embodiments of this invention may be made without departing from the spirit and scope thereof, it is to be understood that this invention is not limited to the specific embodiments thereof except as defined in the appended claims.

**Claims**

1. A deletion-type mutein of acidic fibroblast growth factor (aFGF) which comprises a continuous polypeptide from 90 to 133 amino acid residues of acidic fibroblast growth factor (aFGF).

2. The mutein according to Claim 1, wherein the continuous polypeptide has from 120 to 131 amino acid residues of human aFGF.

3. The mutein according to Claim 1, wherein the continuous polypeptide has the amino acid sequence selected from the group consisting of amino acid sequences depicted in FIG. 4, FIG. 8 and FIG. 9, respectively.

4. A recombinant DNA which comprises a base sequence encoding the deletion-type muteins according to any of Claims 1-3.

5. A vector which comprises the recombinant DNA according to Claim 4.

6. A transformant which comprises the vector according to Claim 5.

7. The transformant according to Claim 6, which is selected from the group consisting of micro-organisms having characteristics essentially identical with either E. coli MM294(DE3)/ pLysS, pTB 1070 (FERM BP-2601), E. coli MM294(DE3)/pLysS, pTB 1209 (FERM BP- 3039) or E. coli MM294(DE3)/ pLysS, pTB 1210 (FERM BP-3040).

8. A process for the preparation of the deletion-type muteins according to any of Claims 1-3, which comprises culturing the transformant according to Claim 6 or Claim 7 in a medium under conditions suitable for the expression of the muteins, and collecting and isolating the mutein from the culture.

9. A process for the production of the recombinant DNA according to Claim 4, which comprises hybridizing an oligonucleotide primer having a mutation, with a single-stranded, structural DNA of aFGF, elongating the primer DNA by DNA polymerase to produce a heteroduplex with the mutation, and replicating the heteroduplex with the mutation to isolate the recombinant DNA.

10. A process for the production of the transformant according to Claim 6 or Claim 7, which comprises inserting the recombinant DNA according to Claim 4 into a suitable vehicle to produce a vector, and transforming a host cell with the vector to obtain the transformant.

Claims for the following Contracting States: ES, GR

1. A process for the preparation of a deletion-type muteins of acidic fibroblast growth factor (aFGF) having a continuous polypeptide selected from the group consisting of from 90 to 133 amino acid residues of aFGF, which comprises culturing a transformant which comprises a recombinant DNA having a base sequence coding for said deletion-type mutein, in a medium under conditions suitable for the expression of the muteins and collecting and isolating the mute in from the culture.

2. The process according to Claim 1, wherein the mutein has an amino acid sequence selected from the group consisting of from 120 to 131 amino acid residues of human aFGF.

3. The process according to Claim 1, wherein the mutein is selected from the group consisting of amino acid sequences depicted in FIG. 4, FIG. 8 and FIG. 9, respectively.

4. A process for the production of a transformant which comprises a recombinant DNA having a base sequence coding for said deletion-type mutein , which comprises inserting said recombinant DNA into a suitable vehicle to produce a vector, and transforming a host cell with the vector to obtain the transformant.

5. The process according to Claim 4, wherein the transformant is selected from the group consisting of micro-organisms having characteristics essentially identical with either E. coli MM294(DE3)/ pLysS, pTB 1070 (FERM BP-2601), E. coli MM294(DE3)/pLysS, pTB 1209 (FERM BP-3039) or E. coli MM294(DE3)/ pLysS, pTB 1210 (FERM BP-3040).

6. The process according to Claim 4, wherein the vector has control sequences.

7. The process according to Claim 4, wherein the vector is replicable.

8. A process for the production of a recombinant DNA having a base sequence coding for a deletion-type mutein which has a continuous polypeptide selected from the group consisting of from 90 to 133 amino acid residues of aFGF, which comprises hybridizing an oligonucleotide primer having a mutation, with a single-stranded, structural DNA of aFGF, elongating the primer DNA by DNA polymerase to produce a heteroduplex with the mutation, and replicating the heteroduplex with the mutation to isolate the recombinant DNA.

9. The process according to Claim 8, wherein the mutein has an amino acid sequence selected from the group consisting of from 120 to 131 amino acid residues of human aFGF.

10. The process according to Claim 8, wherein the mutein is selected from the group consisting of amino acid sequences depicted in FIG. 4, FIG. 8 and FIG. 9, respectively.

```
HindIII              BspMI
   |                   |
   ↓       10          ↓   20          30          40          50          60
AAGCTTACCT GCCATGTTTA ATCTGCCTCC CGGGAATTAC AAGAAGCCCA AACTCCTCTA


        70          80          90         100         110         120
CTGCAGCAAC GGGGGCCACT TCCTGAGGAT TCTTCCGGAT GGCACAGTGG ATGGGACAAG


       130         140         150         160         170         180
GGACAGGAGC GACCAGCACA TTCAGCTGCA ACTCAGTGCG GAAAGCGTGG GGGAGGTGTA


       190         200         210         220         230         240
TATAAAGAGT ACCGAGACTG GCCAGTACTT GGCAATGGAC ACCGACGGGC TTTTATACGG


       250         260         270         280    ·    ·290         300
CTCACAGACA CCAAATGAGG AATGTTTGTT CCTGGAAAGG CTGGAGGAGA ACCATTACAA


       310         320         330         340         350         360
CACCTATATA TCCAAGAAGC ATGCAGAGAA GAATTGGTTT GTTGGCCTCA AGAAGAATGG


       370         380         390         400         410         420
GAGCTGCAAA CGCGGTCCTC GGACTCACTA TGGCCAGAAA GCAATCTTGT TTCTCCCCCT


       430         440         450         460         470         480
GCCAGTCTCT TCTGATTAAT AAGGATCCGA ATTC
                             ↑          ↑
                          BamHI      EcoRI
```

Figure 1

Figure 2

Figure 3

15

M  L  Q  L  S  A  E  S  V  G  E  V  Y  I  K  S  T  E  T  G

Q  Y  L  A  M  D  T  D  G  L  L  Y  G  S  Q  T  P  N  E  E

C  L  F  L  E  R  L  E  E  N  H  Y  N  T  Y  I  S  K  K  H

A  E  K  N  W  F  V  G  L  K  K  N  G  S  C  K  R  G  P  R

T  H  Y  G  Q  K  A  I  L  F  L  P  L  P  V  S  S  D

Figure 4

EP 0 420 222 A1

Figure 5

Figure 6

Figure 7

M K P K L L Y C S N G G H F L R I L P D

G T V D G T R D R S D Q H I Q L Q L S A

E S V G E V Y I K S T E T G Q Y L A M D

T D G L L Y G S Q T P N E E C L F L E R

L E E N H Y N T Y I S K K H A E K N W F

V G L K K N G S C K R G P R T H Y G Q K

A I L F L P L P V S S D

Figure 8

EP 0 420 222 A1

EP 0 420 222 A1

M L L Y C S N G G H F L R I L P D G T V

D G T R D R S D Q H I Q L Q L S A E S V

G E V Y I K S T E T G Q Y L A M D T D G

L L Y G S Q T P N E E C L F L E R L E E

N H Y N T Y I S K K H A E K N W F V G L

K K N G S C K R G P R T H Y G Q K A I L

F L P L P V S S D

Figure 9

European
Patent Office

EUROPEAN SEARCH
REPORT

Application Number

EP 90 11 8518

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,A | WO-A-8 900 198 (BIOTECHNOLOGY RESEARCH ASSOCIATES, J.V.) * page 13, lines 2 - 7 * * page 32, line 22 - page 33, line 15 * * page 62, line 1 - page 64, line 13 * <br> – – – | 9-10,1-8 | C 12 N 15/12 <br> C 12 P 21/02 <br> C 12 N 1/21 // <br> (C 12 N 1/21 <br> C 12 N |
| X | EP-A-0 326 907 (TAKEDA CHEMICAL INDUSTRIES, LTD) * page 6, lines 3 - 49 * <br> – – – | 9-10 | C 12 R 1:19 ) |
| A | FASEB JOURNAL vol. 1, no. 6, December 1987, BETHESDA, MD US pages 434 - 440; THOMAS, K.A.: "Fibroblast growth factors" * the whole document * <br> – – – – – | 1 | |

|  |  |
|---|---|
|  | TECHNICAL FIELDS SEARCHED (Int. Cl.5) |
|  | C 12 N <br> C 12 P |

The present search report has been drawn up for all claims

| Place of search | Date of completion of search | Examiner |
|---|---|---|
| The Hague | 18 December 90 | ANDRES S.M. |